# FASCICULE DE BREVET EUROPEEN

(11) **EP 1 200 609 B1**
(45) Date de publication et mention de la délivrance du brevet: **16.03.2005**
(21) Numéro de dépôt: 00958605.8
(22) Date de dépôt: 25.07.2000
(51) Int. Cl.: C12N 15/63, C12N 15/82, C12N 5/10, A01H 5/00, A01H 5/10

(54) **PROCEDE D'OBTENTION DE LIGNEES ISOTRANSGENIQUES**
VERFAHREN ZUR HERSTELLUNG VON ISOTRANSGENEN ZELLINIEN
METHOD FOR OBTAINING ISOGENIC TRANSGENIC LINES

(30) Priorité: 28.07.1999 FR 9909990
(43) Date de publication de la demande: 02.05.2002
(60) Demande divisionnaire: 04106292.8
(73) Titulaire: Biogemma, 75001 Paris (FR)
(72) Inventeur: PEREZ, Pascual, F-63450 Chanonat (FR); GARCIA, Denise, F-63450 Le Crest (FR)
(74) Mandataire: Flesselles, Bruno F.G.
(86) Numéro de dépôt international: PCT/FR2000/002130
(87) Numéro de publication internationale: WO 2001/007632

(56) Documents cités:
- WO-A-98/32326
- ISHIDA Y ET AL: "HIGH EFFICIENCY TRANSFORMATION OF MAIZE (ZEA MAYS L.) MEDIATED BTY AGROBACTERIUM TUMEFACIENS" BIO/TECHNOLOGY,US,NATURE PUBLISHING CO. NEW YORK, vol. 14, no. 6, 1 juin 1996 (1996-06-01), pages 745-750, XP002046364 ISSN: 0733-222X cité dans la demande
- CHYI ET AL.: "Locations and stability of Agrobacterium mediated Ti plasmid insertions in the lycopersicon genome" MOLECULAR & GENERAL GENETICS, vol. 204, 1986, pages 64-69, XP000920599
- UMBECK ET AL.: "Inheritance and expression of genes for kanamycin and chloramphenicol resistance in transgenic cotton plants" CROP SCIENCE, vol. 29, 1989, pages 196-201, XP000920567

## Description

L'invention concerne un procédé d'obtention de lignées isottansgéniques caractérisé en ce qu'il comprend une étape permettant de cibler le génome receveur d'un ADN-T après transformation d'un hybride.

Par l'expression 'lignées isotransgéniques', on entend des lignées transgéniques isogéniques, l'isogénie étant définie par l'état d'un génotype ne différant d'un autre que par un très petit nombre de gènes (1 ou 2), souvent obtenu par réaoctoisemeut. Les lignées isotransgéniques selon l'invention sont caractérisées en ce qu'elles sont fixées en génotype pur 'lignée d'intérêt' sur l'ensemble du génome et ont intégré de façon stable l'ADN-T contenant le transgène. Elles ont la particularité d'être exemptes de tout fragment provenant de la lignée de transformation et pouvant constituer un fardeau génétique pour les étapes ultérieures de sélection.

Par 'ADN-T' ou ADN de transfert, on entend le fragment d'ADN contenant le gène d'intérêt et les séquences permettant son expression, qui est transféré et intégré dans le génome de l'hôte au cours de la transformation.

On distinguera dans le cadre de l'invention deux types de lignées : les lignées de transformation ou aptes à la transformation. de type A188 par exemple ; et les lignées non aptes à la transformation, nommées ci-après lignées d'intérêt ou lignées agronomiques. Par 'lignées élites', on désignera des lignées agronomiques présentant un potentiel commercial important à une période donnée. Les lignées élites présentent des propriétés agronomiques liées à l'expression de traits de caractère phénotypique relatifs notamment à leur croissance végétative et au rendement, ces propriétés agronomiques étant des caractéristiques techniques marquant leur potentiel commercial, c'est à dire leur aptitude à être employées dans des programmes de sélection variétale pour la mise sur le marché de lignées commerciales.

Le développement d'hybrides commerciaux implique généralement plusieurs étapes : (1) développement de lignées pures homozygotes parentales, à partir de matériel génétique sélectionné sur ses potentialités ; (2) croisement de ces lignées pour l'obtention d'hybrides et (3) évaluation du potentiel commercial de ces hybrides, en fonction des traits de caractères phénotypiques acquis et de leur vigueur hybride ou hétérosis (croissance végétative et rendement). Ce potentiel commercial est d'autant plus grand que les lignées parentales appartiennent à des groupes hétérotiques variés et possèdent des caractéristiques intéressantes. D'où l'importance accordée à la Recherche et au Développement de lignées parentales améliorées, notamment par transgénèse.

Cependant, les techniques de transformation de plantes développées jusqu'ici, ne permettent pas aujourd'hui de transformer directement et efficacement la grande majorité des lignées agronomiques, dont les lignées élites, qui sont récalcitrantes ou non aptes à la transformation (efficacité nulle ou de l'ordre de 1/10 à 1/100), notamment chez le maïs.

De nombreux travaux ont donc porté d'une part, sur l'amélioration des conditions de culture *in vitro* pour les étapes de transformation et régénération et d'autre part, sur la recherche d'un matériel végétal de départ présentant une bonne efficacité de transformation.

Une meilleure connaissance des facteurs environnementaux a ainsi permis d'optimiser les conditions de culture *in vitro* (transformation et régénération) pour une adaptation à un plus grand nombre de génotypes ; mais ces améliorations ne suffisent pas à surmonter la récalcitrance de certains génotypes, notamment ceux d'intérêt agronomique (Armstrong et al., 1992).

Le choix d'un autre matériel végétal que les lignées pures, souvent récalcitrantes à la transformation, a donc été proposé pour la mise au point de procédés, notamment chez le maïs :
1) transformation d'une lignée donneuse apte à la transformation de type A188 (Armstrong et al., 1985) suivie de rétrocroisements successifs avec des lignées pures receveuses non aptes à la transformation, pour obtenir une lignée 'isotransgénique' (au moins 5 à 6 rétrocroisements nécessaires, si l'on veut atteindre l'isogénie parfaite). Dans la pratique, les lignées résultantes de ces rétrocroisements sont au mieux 'pseudo-isogéniques', car un fragment du génome de la lignée donneuse est irrémédiablement lié au transgène. Selon sa taille et sa nature, fonction des processus de recombinaison et/ou de la disponibilité limitée de marqueur moléculaire pour faire le tri, ledit fragment peut constituer un fardeau génétique gênant pour les étapes de sélection ultérieures ; de plus, les phénomènes de recombinaison qui permettraient de réduire ce fardeau sont des événements rares, la recombinaison entre séquences homéologues étant moins efficace qu'entre séquences homologues, rendant obsolètes les gros efforts de rétrocroisements. Il y a donc un risque important de générer des effets génétiques négatifs dans le produit hybride final, via l'utilisation de matériel de départ de type A188 apte à la transformation.
2) transformation directe d'un hybride 'lignée de transformation x lignée d'intérêt agronomique' (Ishida et al., 1996). Celui-ci, associant les aptitudes à la transformation /régénération et les caractéristiques agronomiques de chacune des lignées parentales, peut apparaître comme un matériel végétal de départ plus favorable à la production d'hybrides commerciaux *in fine.* Mais les résultats obtenus par Ishida et al. (1996) montrent que l'efficacité de transformation de l'hybride est bien plus faible que celle obtenue pour la lignée de génotype A188. Par ailleurs, les risques de fardeau génétique dans le produit final ne sont pas évités, puisque le transgène peut s'intégrer sur l'un ou l'autre des chromosomes de l'hybride (c'est-à-dire sur le chromosome lignée donneuse de type A188 dans 50% des cas).

La demande internationale WO 98/32326 (Pioneer) propose de jouer sur les deux paramètres- conditions de culture in vitro et matériel végétal- pour améliorer l'efficacité de transformation d'une part, et rendre d'autre part le procédé de base décrit par Ishida et al. (1996) applicable à d'autres lignées que A188. Cette équipe mentionne une efficacité de transformation meilleure que celle obtenue avec le protocole de base, mais cette efficacité reste encore faible dans le cas des lignées non aptes à la transformation.

On ne disposait donc pas à ce jour de procédé global ni de matériel végétal pour l'obtention de lignées 'isotransgéniques' vraies, intégrant à la fois les besoins en haute fréquence de transformation (excluant l'utilisation de lignées pures non aptes à la transformation) et la nécessité d'avoir une isogénie vraie pour les lignées transgéniques produites (indiquant au contraire l'utilisation de ces lignées pures, pour éviter tout fardeau génétique provenant de la lignée de transformation).

La présente invention permet d'apporter une solution originale à ce problème en mettant au point un nouveau procédé d'obtention de lignées isotransgéniques intégrant une étape qui permet de cibler le génome receveur de l'ADN-T. Ce procédé, basé sur la transformation d'hybride, est en fait caractérisé par une étape de sélection des transformants primaires qui ont intégré uniquement l'ADN-T dans le génome de type non apte à la transformation (*a priori* 50% des transformants). Ces transformants sélectionnés conduiront à la création de lignées isotransgéniques après rétrocroisements desdits transformants avec la lignée d'intérêt agronomique parentale.

Cette étape de sélection des transformants ayant intégré le transgène dans le génome de type non apte à la transformation n'avait jamais été suggérée ni décrite dans l'art antérieur. Elle est avantageuse en ce qu'elle permet d'obtenir *in fine* une lignée isotransgénique 'vraie', c'est-à-dire exempte de tout fragment provenant de la lignée apte à la transformation, tout en gardant un niveau d'efficacité de transformation acceptable. De plus, elle permet d'améliorer la rapidité du transfert du gène d'intérêt dans un génome pur, en diminuant le nombre de rétrocroisements nécessaires.

Le procédé selon l'invention, intégrant cette étape de sélection des transformants primaires, permet de mieux répondre aux exigences industrielles, en matière de rapidité et efficacité, que ne le faisaient les procédés décrits jusqu'à lors.

De plus, ce procédé présente un grand intérêt, notamment lorsque la lignée d'intérêt entre dans de nombreuses formules hybrides ou lorsqu'il s'agit de lignées dominant un marché important. Il permet la production de plantes transgéniques pouvant exprimer, à titre d'exemples, un ARN antisens, un ribozyme ou une protéine d'intérêt lui conférant une résistance aux maladies/pathogènes et/ou une qualité agronomique ou nutritionnelle améliorée (acides aminés, huile, amidon... ).

L'utilisation de ce procédé permet également de varier les sources génétiques des lignées de grands groupes hétérotiques, utilisées comme lignées parentales pour la production d'hybrides commerciaux. Elle rend également possible l'empilement de plusieurs caractères transgéniques dans les lignées agronomiques sans addition de fragments provenant de la lignée de transformation, et pouvant faire l'objet d'un fardeau génétique. Cette perspective intéresse notamment la diversification des sources génétiques pour la production d'hybrides commerciaux ayant conservé une bonne vigueur hybride, voire même améliorée.

Selon un premier mode de réalisation, le procédé d'obtention de lignées isotransgéniques de plantes selon l'invention comprend les étapes suivantes de :
a) transformation des cellules végétales d'un hybride de plante constitué par le croisement de deux lignées parentales, une lignée d'intérêt et une lignée apte à la transformation, avec un vecteur porteur d'un ADN-T contenant un transgène;
b) sélection des transformants primaires hybrides ayant intégré ledit ADN-T uniquement, dans le génome de la lignée d'intérêt ;
c) rétrocroisements avec la lignée parentale d'intérêt desdits transformants primaires sélectionnés en b), et sélection des individus issus de ces rétrocroisements jusqu'à l'obtention de lignées isotransgéniques.

De préférence, parmi les transformants primaires hybrides, sont préalablement choisis ceux qui présentent une insertion monolocus ou monocopie de l'ADN-T, c'est-à-dire ayant intégré de préférence une copie du transgène (monocopie) ou éventuellement plusieurs copies en tandem, au même locus chromosomique. Les individus monocopies sont notamment préférés en ce qu'ils ne sont pas affectés par le phénomène d'extinction de gènes, connu pour les insertions multicopies et qu'ils permettent un suivi simplifié du transgène. Par l'expression 'insertion sans séquence extrabordure', on entend des transformants qui ont intégré uniquement l'ADN-T contenant le transgène, sans le transfert de séquences plasmidiques extérieures à l'ADN-T, nommées extrabordures.

La sélection des transformants monolocus, monocopie de préférence, et dépourvus de séquence extrabordure, peut notamment être réalisée par la technique Southern avec plusieurs enzymes de restriction et plusieurs sondes (Southern, 1975), permettant d'identifier et caractériser l'insertion dans le génome de la plante, et de différencier ainsi les événements de transformation.

Le procédé est caractérisé en ce que l'étape de sélection des transformants primaires hybrides consiste à identifier les séquences génomiques adjacentes à l'ADN-T inséré pour déterminer le génome parent receveur dudit ADN-T.

Pour chaque transformant primaire qui s'est révélé conforme au phénotype attendu et qui a été sélectionné suivant les critères- monolocus ou monocopie et absence d'extrabordures- les séquences génomiques de l'hôte adjacentes à l'ADN-T peuvent être isolées et identifiées, par exemple via une méthodologie basée sur la PCR (Polymerase Chain Réaction, Saiki Rk. et al., 1988), de préférence IPCR (Inverse PCR, Does Mp. Et al., 1991). Le but étant d'identifier l'origine parentale du génome accepteur du transgène (lignée d'intérêt agronomique ou lignée de transformation).

Enfin l'identification, pour chaque transformant, du génome de la lignée parentale ayant intégré l'ADN-T, peut notamment être basée sur la mise en évidence d'un polymorphisme de la taille des fragments de restriction (RFLP, Restriction Fragment Length Polymorphism, Burr B. et al., 1983) entre les lignées parentales et le transformant, en utilisant comme sondes, le ou les fragments génomiques adjacents, précédemment identifiés.

De façon alternative, le séquençage des bordures génomiques de l'ADN-T et la mise en évidence de SNP (Single Nucleotid Polymorphism) par comparaison avec les séquences des lignées parentales, peuvent également permettre l'identification du génome parent receveur.

De plus, lesdites séquences génomiques adjacentes identifiées peuvent encore être utilisées comme sondes sur une population de cartographie connue de l'homme de l'art, pour identifier le chromosome porteur de l'insertion et la position de celle-ci, selon les techniques de cartographie (par exemple Murigneux et al., 1993). Ceci permet de choisir quelques marqueurs autour de cette position, à utiliser avantageusement dans les étapes ultérieures de sélection des individus backcrossés.

La construction de vecteurs d'expression pour la transformation (étape a) est à la portée de l'homme du métier suivant les techniques standards, comme décrit par exemple dans Sambrook et al. (1989). Lesdits vecteurs d'expression peuvent contenir une séquence nucléotidique en sens ou en antisens codant par exemple pour une protéine d'intérêt (qualité agronomique, nutritionnelle ou thérapeutique), ou protéine de résistance à des maladies et/ou pathogènes (herbicide, insecticide), ou un marqueur de sélection, ou un ARN antisens ou un ribozyme..., ainsi que des séquences régulatrices permettant son expression chez la plante (promoteur- constitutif ou inductible ou spécifique/ peptide adressage/ terminateur). Weising et al. (1988) décrit notamment des promoteurs, séquences de polyadénylation, gènes marqueurs de sélection, gènes reporteurs, enhancers, introns utilisables dans le cadre de l'invention. Parmi les séquences nucléotidiques d'intérêt, on peut citer tous les acides nucléiques permettant de donner ou améliorer un trait de caractère bénéfique chez la plante transgénique résultante. Par exemple, l'acide nucléique peut coder pour des protéines ou des transcrits ARN antisens pour favoriser une augmentation des valeurs mutritionnelles, du rendement, de la résistance aux pathogènes, aux maladies.... De tels gènes sont notamment décrits dans les demandes de brevet WO 91/02071 et WO 95/06128.

A titre d'exemple, on peut citer :
- le gène bactérien dapA pour augmenter le taux de lysine ;
- le gène de l'endotoxine Bt ou d'un inhibiteur de protéase ou de protéines extraites de bactéries comme Photorabus (WO 97/17432 & WO 98/08932) pour la résistance aux insectes ;
- parmi les protéines ou peptides d'intérêt conférant de noûvelles prôpriétés de résistance aux maladies on citera notamment les chitinases (WO92/01792), les glucanases (WO 93/02197), l'oxalate oxydase (WO 94/13790), ou encore les peptides antibactériens et/ou antifongiques, en particulier les peptides de moins de 100 acides aminés riches en cystéines comme les thionines ou défensines de plantes, et plus particulièrement les peptides lytiques de toutes origines comprenant un ou plusieurs ponts disulfures entre les cystéines et des régions comprenant des acides aminés basiques, notamment les peptides lytiques suivants : l'androctonine (WO 97/30082 et WO 99/09189), la drosomicine (WO 99/02717), la thanatine (WO 99/24594) ou l'héliomicine (WO 99/53053). Selon un mode particulier de réalisation de l'invention, la protéine ou peptide d'intérêt est choisi parmi les peptides éliciteurs fongiques, en particulier les élicitines (Kamoun et al., 1993 ; Panabières et al., 1995).
- le gène *bar* ou *pat* conférant une tolérance au bialaphos, un gène bactérien ou végétal codant pour une EPSPS pour la résistance à l'herbicide glyphosate (US 4,940,835, US 5, 188, 642, US 4,971,908, US 5,145,783, US 5,312,910, US5,633,435, US5,627,061, US 5,310,667, WO 97/04103) ; le gène codant pour la glyphosate oxydioréductase (US 5,463,175), un gène bactérien ou végétal codant pour une HPPD native, mutée ou chimère (WO 96/38567, WO 98/02562, WO 99/24585, WO 99/24586) conférant une tolérance aux herbicides ayant pour cible l'HPPD (i.e diketones, isoxazoles, mésotrione, etc) ;
- des gènes impliqués dans les procédés de biosynthèse conduisant à un changement de la qualité des produits de la plante transgénique, tels que les gènes codant pour des enzymes de la biosynthèse ou la dégradation de l'amidon (i.e synthases, enzymes de branchement de l'amidon...) ; gènes codant pour des protéines de stockage du grain (i.e sous-unités de gluténines, gliadines, hordéines) ; gènes liés à la force du grain dans le blé (i.e puroindolines).
- les gènes modifiant la constitution des plantes modifiées, en particulier la teneur et la qualité de certains acides gras essentiels (EP 666 918) ou encore la teneur et la qualité des protéines, en particuliers dans les feuilles et/ou les graines desdites plantes. On citera en particulier les gènes codant pour des protéines enrichies en acides aminés soufrés (Korit, A.A. et al. ; WO 98/20133 ; WO 97/41239 ; WO 95/31554 ; WO 94/20828 ; WO 92/14822). Ces protéines enrichies en acides aminés soufrés auront également pour fonction de piéger et stocker la cystéine et/ou la méthionine excédentaire, permettant d'éviter les problèmes éventuels de toxicité liés à une surproduction de ces acides aminés soufrés en les piégeant. On peut citer également des gènes codant pour des peptides riches en acides aminés soufrés et plus particulièrement en cystéines, les dits peptides ayant également une activité antibactérienne et/ou antifongique. On citera plus particulièrement les défensines de plantes, de même que les peptides lytiques de toute origine, et plus particulièrement les peptides lytiques suivants : l'androctonine (WO 97/30082 et WO 99/09189), la drosomicine (WO 99/02717), la thanatine (WO 99/24594) ou l'héliomicine (WO 99/53053).
- des gènes de la stérilité mâle artificielle (i.e bamase, and PR-glucanase sous contrôle d'un promoteur approprié) peuvent également être utilisées pour la production de semences hybrides.

Les séquences nucléiques d'intérêt peuvent également être introduites en tant qu'outil génétique pour générer des mutants et/ou assister l'identification, le marquage moléculaire ou l'isolation de segments de gènes de plantes. D'autres exemples sont décrits dans Weising et al.

Le vecteur d'expression comprenant la séquence nucléique d'intérêt à introduire dans la plante contiendra généralement un marqueur de sélection ou un gène rapporteur ou les deux, pour faciliter l'identification ou la sélection des cellules transformées. De façon alternative, le marqueur de sélection peut être porté par un second vecteur et utilisé en cotransformation. Ces séquences doivent être flanquées de séquences régulatrices appropriées pour permettre leur expression dans les plantes. Les marqueurs de sélection sont bien connus de l'homme de métier et incluent, par exemple, des gènes de résistance aux antibiotiques et aux herbicides. Des exemples particuliers sont décrits dans Weising et al ou les demandes de brevets EP 242 236, EP 242 246, GB 2 197 653, WO 91/02071, WO 95/06128, WO 96/38567 ou WO 97/04103. Un marqueur de sélection préféré est l'hygromycine B phosphotransferase (hpt), qui peut être dérivée de E. Coli. On peut également citer le gène de l'aminoglycoside phosphotransferase du transposon n5 (AphII) qui code pour la résistance aux antibiotiques kanamycine. neomycine, et G418, ainsi que les gènes qui codent pour la résistance ou la tolérance au glyphosate, bialaphos, methotrexate, imidazolinones, sulfonylurées, bromoxynil, dalapon et dérivés. Les gènes marqueurs de sélection conférant une tolérance aux herbicides présentent également une utilité commerciale dans les plantes transformées résultantes. Le gène rapporteur est généralement un gène qui n'est pas présent ou exprimé dans l'organisme ou tissu receveur et qui code pour une protéine dont l'expression est mise en évidence par des propriétés détectables, comme un changement phénotypique ou une activité enzymatique. Des exemples sont donnés dans Weising et al. Parmi les gènes préférés, on peut citer le gène de la Chloramphenicol Acetyl Transferase (cat) de tn9 de *E. Coli,* le gène de la beta-glucuronidase (gus) au locus uidA de *E. Coli,* le gène de la Green Fluorescent Protein (GFP) de *Aequoria victoria,* et le gène de la luciferase de *Photinus pyralis.*

Les séquences régulatrices incluent également des promoteurs constitutif, inductible, spécifique d'un tissu ou d'un organe, ou spécifique du stade développement et qui peuvent être exprimés dans la cellule végétale. De tels promoteurs sont décrits dans Weising et al.

On peut également citer :
- les séquences régulatrices de l'ADN-T de *A. tumefaciens,* incluant la mannopine synthase, la nopaline synthase, l'octopine synthase
- le promoteur de l'aclcohol dehydrogénase de maïs ;
- les promoteurs induits par la lumière tels que le gène de la petite sous-unité de la ribulose-biphosphate-carboxylase d'une variété d'espèces et le promoteur du gène de la protéine de liaison chlorophylle a/b ;
- les promoteurs d'histone (EP 507 698), éventuellement combinés avec le premier intron de l'actine de riz (WO 99/34005) ;
- le promoteur de l'ubiquitine 1 de maïs (Christensen et al., 1996)
- le promoteur 35S du virus de la mosaïque du chou-fleur, ou le promoteur 19S ou avantageusement le promoteur constitutif double 35S (pd35S), décrits dans l'article de Kay et al., 1987 ;
- le promoteur pCRU du gène de la cruciférine de radis permettant l'expression des séquences associées uniquement dans les semences (ou graines) de la plante transgénique obtenue (Depigny-This et al., 1992) ;
- les promoteurs pGEA 1 et pGEA6 correspondant à la région 5' non codante des gènes de la protéine de réserve de graines, GEA1 et GEA6, respectivement, d'Arabidopsis thaliana (Gaubier et al., 1993) et permettant une expression spécifique dans les graines ;
- le promoteur actine du riz suivi de l'intron actine de riz (pAR-IAR) contenu dans le plasmide pActl-F4 décrit par Mc Elroy et al., 1990 ;
- le promoteur HMWG (High Molecular Weight Glutenin) de blé par Robert et al., 1989 ;
- les promoteurs régulés au cour du développement tels que les promoteurs waxy, zéine ou bronze du maïs;
- les promoteurs spécifiques d'un organe ou d'un stade de développement, tels que le promoteur de l'alpha-tubuline décrit dans US 5,635,618.
- le promoteur du gène de zéine de maïs (Pzéine) permettant l'expression dans l'albumen des semences de maïs (Reina et al., 1990) ;
- le promoteur N d'un clone génomique de maïs, dont le cDNA est référencé dans la publication de Shen et al. (1994).

On peut encore utiliser une séquence de régulation promotrice spécifique de régions ou de tissus particuliers des plantes, et plus particulièrement des promoteurs spécifiques des graines (Datla, R. et al., 1997), notamment les promoteurs de la napine (EP 255 378), de la phaseoline, de la glutenine, de l'héliantinine (WO 92/17580), de l'albumine (WO 98/45460), de l'oélosine (WO 98/45461), de l'ATS1 ou de l'ATS3 (WO 99/20775).

On peut également employer un promoteur inductible avantageusement choisi parmi les promoteurs de phénylalanine ammoniac lyase (PAL), d'HMG-CoA reductase (HMG), de chitinases, de glucanases, d'inhibiteurs de proteinase (PI), de gènes de la famille PRI, de la nopaline synthase (nos) ou du gène vspB (US 5 670 349), le promoteur HMG2 (US 5 670 349), le promoteur de la beta-galactosidase (ABG1) de pomme ou le promoteur de l'amino cyclopropane carboxylate syntase (ACC synthase) de pomme (WO 98/45445).

D'autres éléments tels que les introns, enhancers, séquences de polyadénylation et dérivées, peuvent également être présentes dans la séquence nucléique d'intérêt, pour obtenir améliorer l'expression ou le fonctionnement du gène transformant. A titre d'exemple d'enhancer, l'activateur de translation du virus de la mosaïque du tabac (VMT) décrit dans la demande WO 87/07644, ou du virus etch du tabac (VET) décrit par Carrington & Freed (1990). Parmi les introns utilisables, le premier intron AdhIS de maïs peut être placé entre le promoteur et la séquence codante d'une séquence nucléique d'intérêt. Cet intron, inclus dans une construction génétique, est connu pour augmenter l'expression d'une protéine dans les cellules de maïs (Callis et al., 1987). On peut également utiliser le premier intron du gène shrunken-1 de maïs (Maas et al., 1991), le premier intron du gène de la catalase du pois castor (cat-1) (Ohta et al., 1990) ; le second intron du gène ST-LS1 de la catalase de pomme de terre (Vancanneyt et al., 1990) ; l'intron du virus DSV nain jaune du tabac(Morris et al. 1992) ; l'intron actine -1 (act-1) du riz (McElroy et al. 1990) et l'intron 1 de la triose phosphate isomérase (TPI) (Snowden et al., 1996). Cependant, une expression suffisante peut souvent être obtenue sans intron (Battraw et al., 1990).

Le vecteur d'expression peut aussi comprendre des séquences codant pour un peptide de transit, pour amener la protéine codée par le gène hétérologue dans les chloroplastes des cellules de plante. Ces peptides de transit bien connus de l'homme de métier peuvent inclure les peptides de transit simples ou multiples, obtenus par la combinaison de séquences codant pour au moins deux peptides de transit. Le peptide de transit peut être simple, comme un peptide de transit d'EPSPS (décrit dans le brevet US 5,188,642) ou un peptide de transit de celui de la petite sous-unité de ribulose-biscarboxylase/oxygénase (ssu RuBisCO) d'une plante, éventuellement comprenant quelques acides aminés de la partie N-terminale de la ssu RuBisCO mature (EP 189 707) ou encore un peptide de transit multiple comprenant un premier peptide de transit de plante fusionné à une partie de la séquence N-terminale d'une protéine mature à localisation plastidiale, fusionnée à un deuxième peptide de transit de plante tel que décrit dans le brevet EP 508 909, et plus particulièrement le peptide de transit optimisé comprenant un peptide de transit de la ssu RuBisCO de tournesol fusionné à 22 acides aminés de l'extrémité N-terminale de la ssu RuBisCO de maïs fusionnée au peptide de transit de la ssu RuBisCO de maïs tel que décrit avec sa séquence codante dans le brevet EP 508 909. Un peptide transit préféré est Peptide de Transit Optimisé (OTP) décrit dans le brevet US 5,635,618.

La transformation de cellules végétales de l'hybride peut être réalisée par les techniques connues de l'homme de métier.

On peut citer notamment les méthodes de transfert direct de gènes telles que la microinjection directe dans des embryoides de plante (Neuhaus et Coll., 1987), l'infiltration sous vide (Bechtold et al., 1993) ou l'électroporation (Chupeau et Coll., 1989) ou encore la précipitation directe au moyen de PEG (Schocher et Coll., 1986) ou le bombardement par canon de particules (Fromm M. et al., 1990).

On peut également infecter la plante par une souche bactérienne notamment d'*Agrobacterium.* Selon un mode de réalisation du procédé de l'invention, les cellules végétales sont transformées par un vecteur selon l'invention, ledit hôte cellulaire étant susceptible d'infecter lesdites cellules végétales en permettant l'intégration dans le génome de ces dernières, des séquences d'ADN d'intérêt initialement contenues dans le génome du vecteur susmentionné. Avantageusement, l'hôte cellulaire susmentionné utilisé est *Agrobacterium tumefaciens,* notamment selon la méthode décrite dans l'article d'An et al.(1986), ou encore *Agrobacterium* rhizogenes, notamment selon la méthode décrite dans l'article de Jouanin et al., 1987.

De manière préférentielle, la transformation des cellules végétales est réalisée par le transfert de la région T du plasmide circulaire extra-chromosomique inducteur de tumeurs Ti *d'Agrobacterium tumefaciens,* en utilisant un système binaire (Watson et al). Pour ce faire, deux vecteurs sont construits. Dans un de ces deux vecteurs, la région de l'ADN-T a été éliminée par délétion, à l'exception des bords droits et gauche, un gène marqueur étant inséré entre eux pour permettre la sélection dans les cellules de plantes. L'autre partenaire du système binaire est un plasmide Ti auxiliaire, plasmide modifié qui n'a plus d'ADN-T mais contient toujours les gènes de virulence vir, nécessaires à la transformation de la cellule végétale. Ce plasmide est-maintenu dans *Agrobacterium.*

De manière préférentielle, la transformation de cellules végétales par *Agrobacterium tumefaciens* est réalisée selon le protocole décrit par Ishida et al (1996), notamment à partir d'embryons immatures de 10 jours après la fécondation.

De façon alternative, on peut utiliser la méthode de transformation d'embryons immatures décrite dans la demande internationale WO 98/32326, ou la méthode de transformation des inflorescences de plantes monocotylédones décrite dans la demande de brevet WO 99/67357.

Les deux lignées parentales de l'hybride sont ainsi choisies : pour l'une, sur son aptitude à la transformation (lignée parentale de transformation) et pour l'autre, sur sa polyvalence ou son importance commerciale sur le marché (lignée parentale d'intérêt).

Actuellement, la lignée présentant la meilleure aptitude à la transformation par *Agrobacterium* est la lignée A188 ; c'est celle qui est généralement utilisée pour la production de transformant. Parmi les lignées de transformation et les lignées élites commerciales connues, on peut citer notamment celles décrites par Ishida et al (1996) et Pioneer (WO 98/32326).

Parmi les cellules susceptibles d'être transformées selon le procédé de l'invention, on peut citer à titre d'exemples des cellules de plantes de grandes cultures (maïs, blé, colza, tournesol, pois, soja, orge...) ou des plantes potagères et fleurs.

Les étapes de transformation (a) et sélection (étape b- procédé selon l'invention) décrites précédemment ont permis de sélectionner des transformants qui ont intégré le transgène dans le génome de type non apte à la transformation . Lesdits transformants sélectionnés contiennent 50% du génome de la lignée parentale de transformation et 50% du génome de la lignée parentale agronomique.

La reconversion vers une lignée fixée en génome d'intérêt pur (étape c), passe par des rétrocroisements (backcross) successifs avec la lignée parentale d'intérêt et une sélection des individus obtenus selon la méthode classique d'analyse phénotypique ou préférentiellement la sélection assistée par marqueurs (Hospital et al., 1992).

Cette sélection est basée notamment sur les critères suivants :
(i) variabilité autour du site d'intégration du transgène, avec élimination de tout fragment lié provenant de la lignée donneuse de transformation (sélection d'événements de recombinaison). La recombinaison génétique souhaitée est sélectionnée d'un côté du gène à une génération de rétrocroisement et de l'autre côté à la génération suivante.
(ii) recherche du meilleur ratio génome d'intérêt (rapport du % génome d'intérêt agronomique sur le % du génome global) pour l'ensemble du génome.

L'étape (i) s'avère limitante dans le cas où le transgène est inséré dans un génome de type A 188 par exemple, car il est nécessaire d'éliminer tout fragment provenant de cette lignée de transformation en sélectionnant les événements de recombinaison les plus proches du transgène (événements rares). Cela requiert : d'effectuer les étapes de rétrocroisements sur un grand nombre de plantes pour sélectionner au moins une plante recombinée correctement des deux côtés de l'insertion (2è rétrocroisement ou backcross) ; d'attendre un backcross supplémentaire pour appliquer. sur un nombre suffisant de plantes, une pression de sélection sur l'ensemble du génome. S'il est possible d'obtenir *in fine* des plantes fixées en génome d'intérêt à plus de 99% dès le 4è backcross, ces plantes resteront au mieux pseudoisogéniques au site d'insertion du transgène.

Dans le cas où le transgène est inséré dans un génome de type agronomique (lignée parentale d'intérêt), et que les transformants primaires sont sélectionnés pour cette caractéristique selon l'invention, cette étape (i) de sélection d'événements rares de recombinaison n'est plus nécessaire. En conséquence, on obtient une réduction potentielle du nombre de rétrocroisements nécessaires et/ou du nombre d'individus à tester et/ou du nombre de marqueurs pour la sélection, ainsi qu'il sera décrit à l'exemple 4. L'allégement du procédé global de reconversion vers le génome d'intérêt pur s'ajoute au bénéfice majeur de l'invention, qui est l'obtention d'une isogénie vraie pour les lignées transgéniques produites.

L'invention a également pour objet un procédé dans lequel sont sélectionnés dès le premier rétrocroisement en c) les individus dont le chromosome receveur de l'ADN-T a conservé un génotype entièrement de type lignée d'intérêt et qui ont un ratio génome d'intérêt sur l'ensemble du génome d'au moins 75%.

Rentre également dans le cadre de l'invention l'utilisation du procédé pour introgresser plusieurs caractères transgéniques dans une plante sans addition de fragments liés au transgène pouvant faire l'objet d'un fardeau génétique.

L'invention concerne également un procédé permettant de cibler le génome parent receveur d'un ADN-T après transformation d'un hybride, comprenant l'identification des séquences génomiques adjacentes à l'ADN-T inséré.

Selon un autre mode de réalisation, le procédé selon l'invention est caractérisé en ce qu'il comprend une étape ultérieure de croisement entre la lignée isotransgénique selon l'invention et une autre lignée d'intérêt, notamment une autre lignée isotransgénique selon l'invention contenant un transgène différent, pour l'obtention d'hybrides commerciaux.

Les figures et exemples ci-après illustrent l'invention sans en limiter la portée.

### LEGENDES DES FIGURES

**Figure 1 :** carte plasmidique d'un construit dérivé de pBIOS273
**Figure 2 :** Mise en évidence par analyse RFLP sur les transformants primaires, du génome parental receveur de l'ADN-T.

### EXEMPLES

La transformation du maïs à titre d'exemple, peut notamment être réalisée selon le protocole de Ishida et al (1996) qui utilise les propriétés naturelles *d'Agrobacrerium tumefaciens* et la stratégie du système binaire (Hiei et al., 1994).

### Exemple 1 : Préparation des vecteurs

Le plasmide superbinaire est le résultat d'une recombinaison homologue entre un vecteur intermédiaire porteur de l'ADN-T contenant le gène d'intérêt et/ou le marqueur de sélection, et le vecteur pSB1 de Japan Tobacco (EP 672 752) qui contient : les gènes virB et virG du plasmide pTiBo542 présent dans la souche supervirulente A281 *d'Agrobacterium tumefaciens* (ATCC 37349) et une région homologue retrouvée dans le vecteur intermédiaire permettant cette recombinaison homologue.

Le vecteur intermédiaire pour l'Introduction du gène d'intérêt est le vecteur pBIOS 273. Ce vecteur a été généré en 2 grandes étapes :
- clonage du fragment BspDI/XhoI (pAct-Bar-terNos) du vecteur pDM 302 (Cao et al., 1992) dans le vecteur pSB12 (Komari T. et al, 1996) digéré par SmaI / BspDI : le vecteur pDM302 est digéré avec l'enzyme XhoI (site unique sur le vecteur), générant ainsi des extrémités cohésives 5' sortantes. Ces extrémités sont rendues franches après traitement à la Klenow. Une seconde digestion est ensuite effectuée avec BspDI (extrémités cohésives). La jonction des sites XhoI 'franc' et SmaI permet de recréer le site de coupure Xhol (en position 2363). Ces différentes étapes permettent un clonage orienté dans pSB12 et le vecteur résultant est appelé pBIOS 272.
- délétion du site XhoI en position 3363 du vecteur pBIOS 272 par digestion partielle avec XhI et action de la DNA Polymerase I large fragment. Le vecteur obtenu, possédant un site unique XhoI, est nommé pBIOS 273.

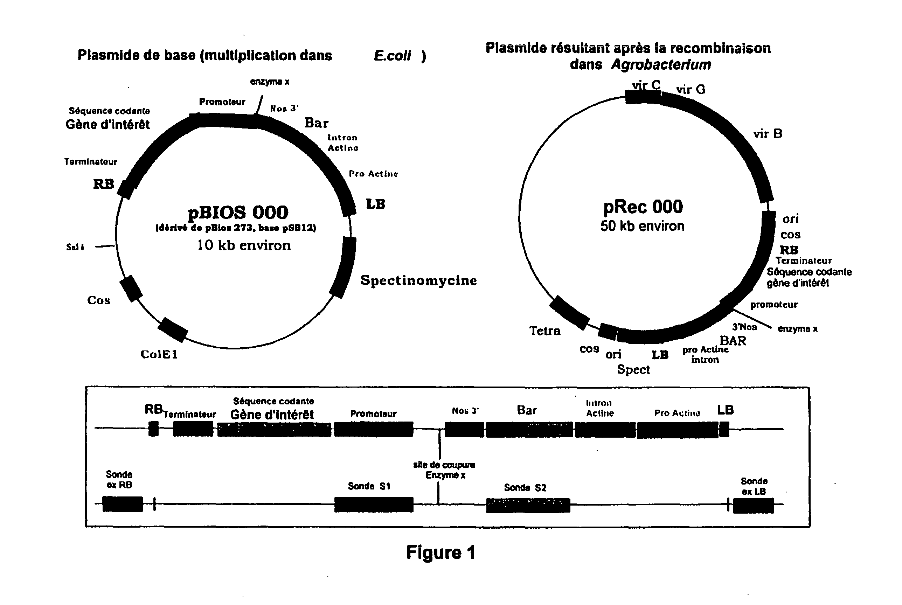

Selon les techniques de clonage bien connues de l'homme de métier, un grand nombre de séquences codant pour un gène d'intérêt peuvent être clonées dans ce vecteur pBIOS 273, aux fins de l'invention (Figure 1).

Le vecteur intermédiaire est introduit dans les cellules *d'A. tumefaciens* souche LBA 4404 (Hoekema et al, 1983) contenant le vecteur pSB 1 par électroporation selon les méthodes bien connues. Les agrobactéries contenant les vecteurs superbinaires sont sélectionnées sur milieu YT CaC12 en présence d'antibiotiques (dont les gènes de résistance sont portés respectivement par les plasmides des différents types), par exemple tétracycline et spectinomycine à une concentration de 50mg/l. Seuls les plasmides superbinaires recombinants porteront la résistance à la spectinomycine (gène initialement sur les plasmides intermédiaires, ne possédant pas d'origine de réplication dans *Agrobacterium,* étant de ce fait incapables de se répliquer dans cette bactérie). Ces plasmides sont ensuite caractérisés par restriction enzymatique et analyse Southern.

### Exemple 2 : Transformation d'hybride de maïs

### a) Obtention de l'hybride

Les lignées choisies pour fabriquer l'hybride à transformer (lignée A188 et lignée d'intérêt) sont semées en serre puis cultivées au phytotron ou en serre après rempotage. Les plantes sont cultivées dans de la tourbe et arrosées quotidiennement avec une solution nutritive SuperPlantora (taux de NPK : 14-10-14 + 3% de MgO). Elles sont soumises à une photopériode de 16 ::8 et à une intensité lumineuse de 3 à 4000 Lux. La température moyenne est de 25°C. Dès l'émergence de l'épi, celui ci est couvert d'un sac en papier pour éviter toute contamination avec du pollen étranger. Ce sac est maintenu jusqu'au moment de la récolte de l'épi.

L'embryon hybride est produit soit en fécondant la lignée A188 avec du pollen de la lignée élite, soit en fécondant la lignée élite avec du pollen de A188.

9 à 10 jours après la fécondation l'épi est observé pour déterminer la taille des embryons. Si celle ci est comprise entre 1 et 1.2 mm, l'épi est récolté et les embryons seront aussitôt prélevés pour être mis en transformation selon le protocole décrit par Ishida et *al.* (1996).

### b) Transformation et régénération

Le protocole de transformation décrit par Ishida et al. (1996) a été choisi dans le cadre de cet exemple ; tous les milieux utilisés sont référencés dans ce protocole. La transformation débute avec une phase de co-culture où les embryons immatures des plantes de maïs sont mis en contact pendant au moins 5 minutes avec *Agrobacterium tumefaciens* LBA 4404 contenant les vecteurs superbinaires. Les embryons sont ensuite placés sur milieu LSAs pendant 3 jours à l'obscurité et à 25°C. Une première sélection est effectuée sur les cals transformés : les 'embryons-cals' sont transférés sur milieu LSD5 contenant de la phosphinotricine à 5 mg/l et de la céfotaxime à 250 mg/l (élimination ou limitation de la contamination par *Agrobacterium tumefaciens*). Cette étape est menée 2 semaines à l'obscurité et à 25°C. La deuxième étape de sélection est réalisée par transfert des embryons qui se sont développés sur milieu LSD5, sur milieu LSD10 (phosphinotricine à 10 mg/l) en présence de céfotaxime, pendant 3 semaines dans les mêmes conditions que précédemment. La troisième étape de sélection consiste à exciser les cals de type I (fragments de 1 à 2 mm) et à les transférer 3 semaines à l'obscurité et à 25°C sur milieu LSD 10 en présence de céfotaxime.

La régénération des plantules est effectuée en excisant les cals de type I qui ont proliféré et en les transférant sur milieu LSZ en présence de phosphinotricine à 5 mg/l et de céfotaxime pendant 2 semaines à 22°C et sous lumière continue.

Les plantules ayant régénéré sont transférées sur milieu RM + G2 contenant 100mg/l d'Augmentin pendant 2 semaines à 22°C et sous illumination continue pour l'étape de développement. Les plantes obtenues sont alors transférées au phytotron en vue de leur acclimatation.

De façon alternative on peut utiliser le protocole décrit dans la demande de brevet WO 98/32326pour transformer des embryons immatures de maïs.

### Exemple 3 : Sélection des transformants qui ont intégré le transgène sur le génome d'intérêt (génome parental non apte à la transformation)

### a) sélection des transformants monocopie et dépourvus de séquence plasmidique indésirable

Parmi les transformants primaires, sont donc préférentiellement choisis ceux qui présentent une insertion monolocus ou monocopie sans séquence plasmidique indésirable. La technique Southern avec plusieurs enzymes de restriction et plusieurs sondes appropriées (Southern, 1975) peut notamment être utilisée pour identifier et caractériser l'insertion dans le génome de la plante, permettant ainsi de différencier les événements de transformation. Cette méthodologie permet en effet de mettre en évidence des différences individuelles dans la taille des fragments de restriction obtenus avec une enzyme donnée et une sonde donnée, correspondant à des emplacements définis sur le génome.

On peut utiliser, à titre d'exemple. le protocole décrit dans Sambrook et al. (1989). L'ADN génomique est extrait à partir de feuilles des transformants primaires suivant un protocole d'extraction au CTAB (Dean C. et al., 1992). Cet ADN est ensuite digéré selon les techniques de biologie moléculaire bien connues, par une enzyme de restriction coupant au moins une fois à l'intérieur de l'ADN-T. A l'aide de sondes appropriées qui s'hybrident de part et d'autre du site de coupure. on peut ainsi caractériser l'insertion des deux côtés internes -bordures droite et gauche - de l'ADN-T suivant la procédure adaptée. Pour une même sonde et pour plusieurs individus, des différences observées dans la taille des bandes reflètent des sites d'insertion différents. Les fragments d'ADN obtenus sont séparés sur un gel d'agarose de 0.9 à 1% puis transférés sur une membrane Hybond N+ (Amersham). L'ADN du plasmide intermédiaire comprenant l'ADN-T porteur du gène d'intérêt et du marqueur de sélection, est inclus dans l'analyse comme témoin. La membrane est hybridée avec des sondes homologues aux séquences du transgène étudié :
- une sonde spécifique du gène marqueur sélectif, dénommée S1.
- une sonde spécifique du gène d'intérêt, dénommée S2.
- deux sondes dénommées ex RB et ex LB (pour extra Right Border et extra Left Border) qui sont utilisées conjointement pour l'hybridation. Cette hybridation nous permet d'éliminer les plantes contenant des séquences externes à l'ADN-T (extra-bordures). Une intégration correcte suppose que seul l'ADN-T est inséré dans le génome de l'hôte, sans séquence extrabordure. Les séquences des plasmides de base étant connues (pBIOS273 dérivé de pSB 12), les sondes ex RB et ex LB sont obtenues par amplification avec des oligonucléotides spécifiques des régions plasmidiques extra-bordures du T-DNA. RB2 et 3 pour ex RB, LB2 et 3 pour ex LB. Les plantes retenues à l'issue de cette analyse moléculaire ne présentent pas de signal d'hybridation avec les sondes ex RB et ex LB, et présentent si possible une seule bande avec chacune des sondes S1 et S2, traduisant une insertion simple monocopie (une copie du gène de sélection et une copie du gène d'intérêt). Suivant la procédure adoptée, des différences dans la taille des bandes entre plantes seront le reflet d'insertions différentes et correspondant à des événements de transformation différents.

### b) identification des séquences génomiques adjacentes à l'ADN-T inséré

Pour chaque transformant primaire qui s'est révélé conforme au phénotype attendu et qui a été sélectionné suivant les critères- monolocus ou monocopie et absence d'extrabordures- les séquences génomiques adjacentes à l'ADN-T peuvent être isolées et identifiées par exemple via une méthodologie basée sur la PCR. Le but étant d'identifier l'origine parentale du génome receveur du transgène (lignée d'intérêt ou lignée de transformation). Plusieurs techniques basées sur la PCR peuvent être utilisées, par exemple le PCR walking (Devic et al., 1997); de préférence, le kit commercial Universal GenomeWalker de la société Clontech peut être utilisé dans le cadre de cette invention. On peut suivre le protocole suivant, basé sur la notice d'utilisation de ce kit : l'ADN des transformants primaires précédemment sélectionnés est digéré séparément par 5 enzymes de restriction (enzymes qui génèrent des fragments d'ADN présentant des extrémités franches) ; les enzymes utilisées peuvent être celles préconisées par le fournisseur à site de restriction 6 paires de bases pb- DraI, Eco RV, Pvull, ScaI et StuI ou d'autres enzymes spécifiques de sites de restriction à 4 ou 5pb. Pour chaque échantillon, les fragments générés sont ensuite liés aux deux extrémités à l'adaptateur GenomeWalker fourni avec le kit. Chaque échantillon est ensuite séparé en deux (ech1 et ech2) pour pouvoir déterminer les séquences génomiques contiguës aux deux bordures de l'ADN-T. La récupération des régions génomiques flanquant les deux bordures de l'ADN-T permet non seulement de confirmer le résultat de l'intégration mais également de faciliter l'identification du parent receveur et de permettre une vérification de la cartographie génétique si nécessaire.

Deux types d'oligonucléotides sont désignés pour la mise en oeuvre des amplifications PCR successives : les oligos AP pour Adaptor Primer, fournis par le kit ; et les oligos GSP pour Gene-Specific Primer, dont le choix dépend de la séquence du vecteur dérivé de pSB12 et des paramètres définis dans la notice d'utilisation du kit. Parmi les oligonucléotides GSP pouvant être utilisés selon l'invention, on peut citer les oligonucléotides identifiés par le logiciel MacVector (version 6) à partir des caractéristiques décrites dans le Tableau ci-dessous.

| **Nom** | **Séquence** | **Taille** | **Tm(°C)** | **position (pHIOS273)** | **% GC** |
|---|---|---|---|---|---|
| **GSPLB1** | ID NO 5 | 29 | 71,3 | 3020 | 58,6 |
| **GSPLB2** | ID NO 6 | 29 | 64,9 | 3081 | 41,4 |
| **GSPLB3** | ID NO 7 | 28 | 70,1 | 3021 | 57,1 |
| **GSPLB4** | ID NO 8 | 27 | 70,1 | 3018 | 59,3 |
| **GSPLB5** | ID NO 9 | 27 | 70,1 | 3019 | 59,3 |
| **GSPLB6** | ID NO 10 | 27 | 68,7 | 3022 | 55,6 |
| **GSPLB7** | ID NO 11 | 27 | 63,3 | 3064 | 40,7 |
| **GSPLB8** | ID NO 12 | 27 | 63,3 | 3077 | 40,7 |
| **GSPLB9** | ID NO 13 | 26 | 68,7 | 3019 | 57,7 |
| **GSPLB11** | ID NO 14 | 26 | 68,7 | 3023 | 57,7 |
| **GSPLB13** | ID NO 15 | 26 | 63,0 | 3078 | 42,3 |
| **GSPRB1** | ID NO 16 | 29 | 67,5 | 571 | 48,3 |
| **GSPRH2** | ID NO 17 | 29 | 67,5 | 592 | 48,3 |
| **GSPRB3** | ID NO 18 | 29 | 67,5 | 655 | 48,3 |
| **GSPRB4** | ID NO 19 | 29 | 66,2 | 656 | 44,8 |
| **GSPRB5** | ID NO 20 | 28 | 67,4 | 570 | 50 |
| **GSPRB6** | ID NO 21 | 28 | 66,1 | 591 | 46,4 |
| **GSPRB7** | ID NO 22 | 28 | 66,1 | 654 | 46,4 |
| **GSPRB8** | ID NO 23 | 28 | 66,1 | 655 | 46,4 |
| **GSPRB9** | ID NO 24 | 27 | 67,4 | 569 | 51,9 |
| **GSPRB10** | ID NO 25 | 27 | 66,0 | 590 | 48,1 |
| **GSPRB11** | ID NO 26 | 27 | 70,1 | 614 | 59,3 |
| **GSPRB12** | ID NO 27 | 27 | 64,6 | 653 | 44,4 |
| **GSPRB13** | ID NO 28 | 27 | 64,6 | 654 | 44,4 |
| **GSPRB14** | ID NO 29 | 26 | 65,9 | 568 | 50 |
| **GSPRB15** | ID NO 30 | 26 | 64,5 | 589 | 46,2 |

Une première amplification PCR, utilisant par exemple les oligos de type GSPLB ou GSPRB selon qu'ils soient spécifiques d'une séquence interne de l'ADN-T côté RB ou LB, est effectuée comme suit :
- avec l'oligo AP1 spécifique de l'adaptateur et l'oligo GSPRBx pour échantillon 1,
- avec l'oligo AP1 et l'oligo GSPLBx pour échantillon2.
Les produits d'amplification sont dilués puis soumis à une deuxième amplification:
- avec l'oligo AP2 spécifique de l'adaptateur et l'oligo GSPRBy pour échantillon 1 ,
- avec l'oligo AP2 et l'oligo GSPLBy pour échantillon2.
Ces oligonucléotides GSP sont utilisables notamment pour tous les vecteurs dérivés de pBIOS273, dans lesquels seule la séquence du gène d'intérêt serait remplacée.
Des séquences spécifiques des gènes insérés à l'intérieur de l'ADN-T peuvent être également utilisées.

Les produits d'amplification PCR obtenus sont analysés sur gel et l'on choisit préférentiellement ceux qui sont supérieurs à 200-300 pb. Ces produits d'amplification PCR - qui contiennent une partie de séquence connue (entre oligo GSP et bordure de l'ADN-T) et une partie de séquence génomique inconnue (entre oligo AP et bordure de l'ADN-T) sont ensuite clonés par exemple dans le vecteur plasmidique pGEM-T (Promega) selon les recommandations du fournisseur puis séquences avec les oligonucléotides universels direct et reverse. Le cas échéant, des oligonucléotides internes pourront être utilisés pour compléter les données. Il est ainsi possible de générer des sondes 'spécifiques' de la séquence génomique de l'hôte bordant l'ADN-T.

### c) Identification du génome parent receveur.

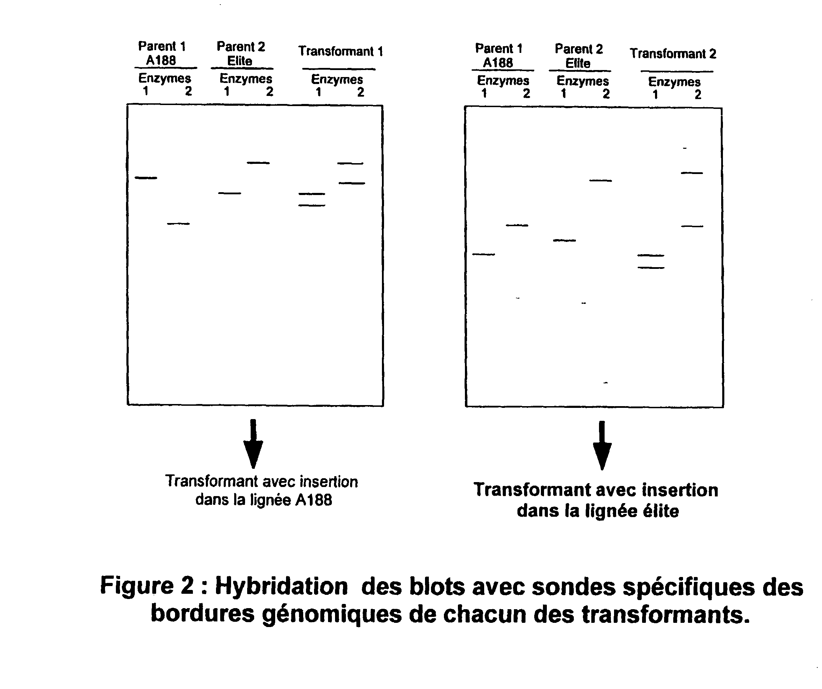

Cette dernière étape conduisant à l'identification, pour chaque transformant, du génome de la lignée parentale ayant intégré l'ADN-T, peut notamment être réalisée selon le protocole suivant (Sambrook et al. 1989). Les bordures récupérées sont utilisées comme sondes et hybridées sur un transfert de gel d'électrophorèse contenant l'ADN digéré séparément par différentes enzymes de restriction des différents transformants et des deux lignées parentales. La mise en évidence d'un polymorphisme de la taille des fragments de restriction (RFLP) homologues à la sonde - et donc au locus d'insertion - entre les lignées parentales, nous permet de définir le parent receveur par comparaison avec le profil du transformant, hétérozygote pour l'insertion. Par l'expression 'hétérozygote (hémizygote) pour l'insertion', on entend que le transformant hybride porte l'ADN-T contenant le transgène sur un seul des deux chromosomes Une intégration dans la lignée parentale d'intérêt se traduit donc par un RFLP par rapport à la lignée d'intérêt et non par rapport à l'autre parent. Dans le cas d'une insertion simple dans la lignée d'intérêt, le profil du transformant primaire se caractérise par deux bandes : une bande de taille identique à celle du parent de transformation et une bande de taille différente de celle du parent d'intérêt. A titre d'exemple la Figure 2 présente les profils attendus pour les parents et le transformant, dans le cas d'une insertion dans le chromosome de l'un ou l'autre des parents (2 cas).

L'identification du parent receveur peut également être obtenue selon une autre alternative, qui consiste à utiliser les données de séquençage des bordures génomiques de l'ADN-T obtenues en b) pour mettre en évidence des SNP (Single Nucleotide Polymorphism) entre les lignées parentales. Après clonage dans pGEM-T de la séquence adjacente génomique récupérée et détermination de la séquence complète, des oligonucléotides peuvent être désignés sur la séquence et utilisés pour de nouvelles amplifications PCR sur les lignées parentales et le transformant. S'il existe un polymorphisme nucléotidique entre les deux parents pour la portion génomique ayant servi à désigner les oligonucléotides, une insertion dans la lignée d'intérêt se traduira par une amplification pour la lignée d'intérêt et le transformant et pas d'amplification pour l'autre parent. Si par contre, aucun polymorphisme n'est détecté (amplification d'un même fragment chez les deux lignées parentales, dans le cas d'un fragment génomique conservé), le séquençage desdits fragments amplifiés chez les lignées parentales sera nécessaire. L'analyse comparative avec les séquences adjacentes génomiques du transformant, identifiées et séquencées en b), déterminera alors le parent receveur du transgène. Selon l'une ou l'autre méthode, il sera possible de différencier les lignées pseudo-isogéniques obtenues par les procédés décrits dans l'art antérieur des lignées isotransgéniques vraies obtenues selon l'invention.

### Exemple 4 : Rétrocroisements avec le parent d'intérêt et sélection des individus jusqu'à l'obtention de lignées isotrangéniques.

Les étapes précédentes ont permis de sélectionner des transformants qui ont intégré le transgène dans le génome d'intérêt; par ailleurs lesdits transformants contiennent 50% en génome parent de transformation et 50% en génome d'intérêt.

Préférentiellement, la sélection des plantes issues des rétrocroisements avec la lignée parentale d'intérêt, est assistée par marqueurs selon les méthodes connues, notamment celle décrite par Ragot et al. (1995).

A titre comparatif et démonstratif des avantages de la sélection des transformants primaires selon l'invention, sera décrit en préambule le cas d'un processus de sélection après insertion du transgène dans le génome de type A188.

### Cas d'une insertion sur un chromosome de type A188 (pas de sélection des transformants primaires)

Classiquement, la recombinaison génétique souhaitée est sélectionnée d'un côté du gène à une génération de rétrocroisement et de l'autre côté à la génération suivante. La taille du génome du maïs étant estimée à 2000 centimorgans (cM), les événements de recombinaisons à sélectionner lors des deux premiers rétrocroisements (BC1 et BC2), pour ne transférer que 1/1000^{ième} du génome non-élite lié au transgène, devront se situer à 1cM de part et d'autre de l'insertion, respectivement.

La sélection des événements de recombinaison assistée par des marqueurs prédéfinis (proches du transgène) est effectuée en BC1 et BC2 sur un nombre de plantes calculé comme suit : le nombre N de plantes à tester pour obtenir 1 plante recombinée à 1cM avec une probabilité de 95%, est N= (log0,05) / (log(1-0,01)) ~300 plantes, selon la loi de probabilité bien connue. Sachant qu'une seule plante recombinée est obtenue à l'issue de ces 2 rétrocroisements, il paraît difficile d'appliquer de surcroît une sélection pour un ratio génome d'intérêt optimal. Les ratios génome d'intérêt attendus en BC1 et BC2 sont en moyenne de 75% et 87,5% respectivement. Une pression de sélection sur l'ensemble du génome ne pourra véritablement être appliquée qu'en BC3, avec une centaine de marqueurs répartis sur l'ensemble du génome de maïs (banque de donnée de l'Université du Missouri)..

La reconversion en génome d'intérêt, pour ce cas particulier, nécessite d'effectuer les étapes de rétrocroisements sur un grand nombre de plantes (sélection d'événements rares sur le chromosome porteur) et d'attendre au moins le 3è backcross pour exercer la seconde pression de sélection, sur l'ensemble du génome. Enfin, les plantes obtenues *in fine* restent au mieux pseudo-isogéniques (fardeau génétique potentiel).

| Backcross | Sélection événement recombinaison au site d'insertion du transgène | | Caractérisation de l'ensemble génome pour le génotype d'intérêt | |
|---|---|---|---|---|
| | Nb plantes testées | Plante avec bonne recombinaison | Nb marqueurs à tester sur génome hétérozygote résiduel | % génome d'intérêt |
| BC1 | **300** | 1 | 100 | 75 |
| BC2 | 300 | 1 | 50 | 87,5 |
| BC3 | 100 | | 25 | 96,8 |
| BC4 | 100 | | 7 | 99,2 |
| Nb total tests : (300x1)+100+(300x1)+50+(100x25)+(100x7)=**3950 tests**, pour sélectionner **1 plante** qui soit **pseudoisogénique** au site du transgène (porte 1/1000 du génome A188 lié au transgène soit en moyenne 50 à 80 gènes, le génome de maïs possédant en moyenne 50000 à 80000 gènes) et **fixée à plus de 99% en génome d'intérêt en BC4.** | | | | |

### Cas d'une insertion sur un chromosome de la lignée parentale d'intérêt (sélection selon l'invention du transformant primaire correspondant, avant rétrocroisements avec le parent d'intérêt).

Selon les orientations choisies en fonction des priorités accordées à la biotechnologie, et/ou la production/rendement et/ou la sélection, plusieurs schémas de sélection sont possibles, intégrant le cas échéant une présélection sur le chromosome receveur de l'insertion. Contrairement au cas d'une insertion dans un génome de type A188, on sélectionnera ici des événements de recombinaison situés loin du transgène ou aucune recombinaison sur le chromosome receveur du transgène.
A titre d'exemples, on peut citer les options suivantes.

### Option 1 : Pas de sélection au site d'intégration du transgène ; sélection ratio génome d'intérêt dès BC1.

| Backcross | Sélection événement recombinaison au site d'insertion du transgène | | Caractérisation de l'ensemble génome pour le génotype d'intérêt | |
|---|---|---|---|---|
| | Nb plantes testées | Plante avec bonne recombinaison | Nb marqueurs | % génome d'intérêt |
| BC1 | **100** | | 100 | 87,5 |
| BC2 | 100 | | 25 | ≈ 100 |
| BC3 | 100 | | 7 | ≈ 100 |
| Nb total tests : (100x100)+(100x25)+(100x7)=**13200 tests**, pour sélectionner **1 plante isogénique vraie** au site du transgène et **fixée à ≈ 100% en génome d'intérêt en BC3** (gain de un rétrocroisement). | | | | |

### Option 2: Sélection en BC1 de l'individu ayant le chromosome porteur du transgène qui soit entièrement de type lignée d'intérêt ; sélection en BC3 pour le ratio génome d'intérêt.

Connaissant le chromosome sur lequel est inséré l'ADN-T (cartographie), il est possible de sélectionner en BC1, à l'aide de 10 marqueurs répartis sur ce chromosome, une plante pour laquelle l'intégrité du chromosome receveur d'intérêt est conservée (aucun événement de recombinaison). Sachant que les chromosomes font une longueur moyenne de 200cM, la probabilité d'avoir une plante sans événement de recombinaison est P=(0,99)²⁰⁰~0,14. Le nombre N de plantes à tester avec une probabilité de 95% de l'obtenir est N= (log0,05) / (log(1-0,14)) ~20.

La plante ainsi sélectionnée sera backcrossée avec le parent d'intérêt jusqu'à la reconversion totale (100%) en génome d'intérêt sur l'ensemble du génome.

| Backcross | Sélection de l'intégrité génome élite sur le chromosome porteur du transgène, (10 marqueurs) | | Caractérisation de l'ensemble génome pour le génotype d'intérêt | |
|---|---|---|---|---|
| | Nb plantes testées | Plante avec chromosome élite | Nb marqueurs | % génome d'intérêt |
| BC1 | **20** | 1 | | 75 |
| BC2 | 100 | | | 87,5 |
| BC3 | 100 | | 25 | ≈100 |
| BC4 | 100 | | 7 | ≈ 100 |
| Nb total tests : (20x10)+(100x25)+(100x7)=**3400 tests** pour sélectionner **1 plante isogénique vraie** au site du transeène et **fixée à ≈ 100% en génome d'intérêt dès BC3.** | | | | |

Selon les schémas, on recherchera une réduction du nombre de backcross (rapidité de production) ou une réduction du nombre de plantes à utiliser pour les rétrocroisements, en plus de l'isogénie obtenue de fait selon le procédé de l'invention.

### Exemple 5 : Production d'hybrides commerciaux

Selon les techniques connues de l'homme de métier et ses connaissances en matière de floraison pour chaque lignée élite parentale (Gallais A. et al., 1983), il est possible de croiser lesdites lignées isotransgéniques obtenues à l'exemple 4, en vue d'obtenir des hybrides commerciaux.

### Exemple 6 : Détermination du génome d'insertion du transgène

13 événements ont été obtenus après transformation d'embryons hybrides selon le protocole décrit précédemment à l'exemple 2. Ces événements ont ensuite été analysés par Southern, pour déterminer le nombre de copies du transgène intégré, comme décrit précédemment à l'exemple 3 (a). 3 de ces événements se sont avérés être monocopie.

Le transformant 152-2E, choisi pour la récupération des bordures génomiques décrite ci-dessous, a été obtenu après transformation avec le plasmide superbinaire recombinant pRec 290 issu de pBIOS 290, dérivé de pBIOS 273 par l'intégration au site Xhol d'une cassette « Pro HMWG-PhytI-Nos 3' - Pro HMWG-PhytII-Nos 3' ». Ladite cassette est obtenue selon les techniques de clonage classiques, à partir de la séquence Pro HMWG de blé (Roberts et al The Plant Cell. 1 :569-578, 1989), des séquences nucléotidiques PhytI (N° d'accès EMBL, GenBank : AJ223470) et PhytII (N° d'accès EMBL, GenBank : AJ22347I ), et de la séquence Nos3' (Depicker et al., Mol. Gen. Genet. 235 (2-3) : 389-396, 1992) et des enzymes de restriction appropriées.

La récupération des bordures génomiques a été réalisée du côté bordure droite (RB) par la technique de PCR ancrée en utilisant le kit Genome Walker (Clontech laboratories inc.,Palo Alto, California). Comme décrit à l'exemple 3 (b), l'identification des séquences génomiques adjacentes à l'ADN-T inséré comprend les étapes suivantes :

Le couple d'oligonucléotides GSPRB3/AP1 a permis de réaliser la première PCR sur de l'ADN du transformant 152-1E digéré à EcoRV. Le produit de cette amplification a ensuite été soumis à une deuxième PCR avec le couple d'oligonucléotides GSPRB9/AP2.

Les caractéristiques des oligonucléotides GSPRB3 et GSPRB9 sont décrites dans le tableau de l'exemple 3 et correspondent,aux séquences ID N0 18 et ID N0 24 en annexe.

Les oligonucléotides AP1 et AP2 sont ceux fournis dans le kit Genome Walker et les conditions de PCR sont celles préconisées par le fabricant Clontech. Le fragment bordure de 380 pb obtenu à cette dernière étape a été cloné dans le vecteur pGEMT (Promega corporation, Madison, Wisconsin), pour être amplifié et utilisé comme sonde.

L'identification du génome receveur décrit à l'exemple 3 (c), à partir du fragment bordure récupéré, consiste à hybrider ce fragment sonde sur un transfert de gel d'électrophorèse contenant l'ADN du transformant 152-2E digéré avec EcoRV ainsi que l'ADN des 2 lignées parentales de l'hybride (A188 et L2) et celui de 6 autres transformants.

Le résultat de l'hybridation est représenté sur la figure 3 : sur cette autoradiographie, on visualise de l'ADN correspondant à 7 transformants différents, sachant qu'il y a de 1 à 3 plantes par transformant.

L'hybridation avec la sonde bordure met en évidence 1 bande spécifique au génotype A188 (1.7Kb) et 1 spécifique de la lignée élite L2 (2.5 Kb). On retrouve ces 2 bandes chez tous les transformants (prouvant bien qu'ils sont issus de l'hybride) sauf pour l'événement t152-1E, qui lui présente une bande plus basse à 0.8 Kb environ. On en déduit que le transgène s'est inséré dans le fragment attendu EcoRV de 1.7 Kb du génome de la lignée A 188.

Cette expérience confirme donc la possibilité d'identifier, selon le protocole décrit, le génome d'insertion de l'ADN-T dans le cas de transformant produit par la technique de transformation d'hybride, génome A188 dans ce cas précis.

Selon le même protocole, il est également possible d'obtenir des transformants pour lesquels l'insertion de l'ADN-T s'effectue dans le génome élite, avec une probabilité de 1 transformant sur 2 en moyenne.

Dans le cas où le génome du parent élite est identifié comme le génome receveur de l'insertion de l'ADN-T, des rétrocroisements peuvent être réalisés avec le parent d'intérêt et testés en sélection jusqu'à l'obtention de lignées isotransgéniques, comme décrit précédemment à l'exemple 4.

### BIBLIOGRAPHIE

An et al, Plant Physiol., 81 : 86-91, 1986.

Armstrong, C.L. et al., Maize Genet. Coop. News Letter 59:92-93, 1985.

Armstrong C.L. et al., Theor Appl Genet 84 :755-762, 1992.

Battraw et al., Plant Mol. Biol., 15 :527, 1990.

Bechtold N. et al., Comptes rendus Académie des Sciences Paris Série 3, 316 : 1194-1199, 1993.

Burr B. et al., Genetic Engineering : principles and methods. Setlow A, Hollaender (eds.) Plenum press NY 5 : 45-59, 1983.

Cao et al., Plant Cell Reports : 11 : 586-591,1992.

Callis et al., Genes Dev., 1 :1183, 1987.

Carrington J.C. et Freed D.D, Journal of Virology, 64(4) : 1590-1597,1990.

Chupeau et al., Biotechnology, 7(5) : 503-508, 1989.

Christensen et al., Transgenic Res., 5 : 213, 1996.

Datla R. et al., Biotechnology Ann. Rev., 3 :269-296, 1997.

Dean C. et al., Plant Journal, 2, 69-81, 1992.

Depigny-This et al., Plant. Mol. Biol 20 : 467-479, 1992.

Dévie et al., Plant Physiol and Biochem., 35(4) : 331-33, 1997.

Does Mp et al., Plant. Mol. Biol., 17(1) : 151-3, 1991.

Fromm M. et al., Biotechnology, 8 : 833-839, 1990.

Gallais A. et al., Agromaïs, 20, 40, 1983.

Gaubier et al., Mol. Gen. Genet., 238 :409-418, 1993.

Hiei et al., The plant Journal 6 : 271-282, 1994.

Hospital et al., Genetics, 132 : 1199-1210, 1992.

Hoekema et al, Nature, 303, 179-180, 1983.

Ishida et al., Nature Biotechnology, 14 :745-750, 1996.

Jouanin et al., Plant. Sci., 53 : 53-63, 1987.

Kamoun S. et al., Mol Plant Microbe Interact, 6(5) :573-81; Sept-Oct 1993.

Kay et al., Science 236 : 1299-1302. 1987.

Komari T. et al., The Plant Journal, 10(1) : 165-174, 1996.

Korit A.A et al., Eur. J. Biochem., 195, 329-334,1991.

Maas et al., Plant Mol. Biol., 16 : 199, 1991.

McElroy et al., Plant Cell, 2 : 163-171, 1990.

Morris et al., Virology, 187 :633, 1992.

Murigneux et al., Theor. Appl. Genet. 87 : 278-287, 1993.

Neuhaus G. et al., Theoretical and Applied Genetics, 75(1) : 30-36, 1987.

Ohta et al., Plant Cell Physiol, 31 :805, 1990.

Panabieres F. et al., Mol Plant Microbe Interact, 8(6) : 996-1003, Nov-Dec 1995.

Ragot et al., Techniques et utilisations des marqueurs moléculaires, Les Colloques, n° 72, Ed Reina et al., N.A.R, 18 :6426, I 990

Robert et al., Plant Cell, 1 : 569-578, 1989.

Saiki Rk. et al., Science 29 : 487-491, 1988.

Sambrook et al., Molecular Cloning : a laboratory manual, Cold Spring Harbor laboratory, Press, New York, 1989.

Schocher et al., Biotechnology, 4 : 1093-1096, 1986

Shen et al., Plant. Mol. Biol., 26 : 1085-1101, 1994.

Snowden et al., Plant Mol. Biol., 31 :689, 1996.

Southern, Journal of molecular Biology, 98 : 503-517, 1975.

Vancanneyt et al., Mol Gen. Genet, 220 :245, 1990.

Watson et al., Adn recombinant, Ed. De Boeck université, 273-292.

Weising et al., Annual Rev. Genet, 22 : 241, 1988.

## Revendications

1. Procédé d'obtention de lignées isotransgéniques de plantes, comprenant les étapes suivantes de :
a) transformation des cellules végétales d'un hybride de plante constitué par le croisement de deux lignées parentales, une lignée d'intérêt et une lignée apte à la transformation, avec un vecteur porteur d'un ADN-T, fragment d'ADN contenant un transgène et les séquences permettant son expression, transféré et intégré dans le génome de l'hôte au cours de la transformation;
b) sélection des tranformants primaires hybrides ayant intégré ledit ADN-T uniquement dans le génome de la lignée d'intérêt ;
c) rétrocroisements avec la lignée parentale d'intérêt desdits transformants primaires sélectionnés en b), et sélection des individus issus de ces rétrocroisements jusqu'à l'obtention de lignées isotransgéniques.

2. Procédé selon la revendication 1, **caractérisé en ce que** l'étape de sélection des transformants primaires hybrides consiste à identifier les séquences génomiques adjacentes à l'ADN-T inséré pour déterminer le génome parent receveur dudit ADN-T.

3. Procédé selon la revendication 2, dans lequel la détermination du génome parent receveur dudit ADN-T à partir desdites séquences génomiques adjacentes à l'ADN-T se fait selon une technique de RFLP ou une méthode de séquençage.

4. Procédé selon l'une des revendications 1 à 3, dans lequel sont sélectionnés dès le premier rétrocroisement en c) les individus dont le chromosome receveur de l'ADN-T a conservé un génotype entièrement de type lignée d'intérêt et qui ont un ratio génome d'intérêt sur l'ensemble du génome d'au moins 75%.

5. Procédé selon l'une des revendications précédentes, **caractérisé en ce qu'**il comprend une étape ultérieure de croisement entre la lignée isotransgénique selon l'invention et une autre lignée d'intérêt, notamment une autre lignée isotransgénique contenant un autre transgène, pour l'obtention d'une lignée hybride.

6. Procédé selon l'une des revendications précédentes, **caractérisé en ce que** les cellules végétales proviennent d'une espèce de grande culture choisie parmi le maïs, blé, colza, tournesol, pois, soja, orge ou d'une espèce potagère ou florale.

7. Procédé selon l'une des revendications précédentes, **caractérisé en ce que** l'ADN-T comprend notamment une séquence nucléotidique codant pour une protéine conférant des propriétés agronomiques et/ou de résistance aux maladies.

8. Procédé selon l'une des revendications précédentes, **caractérisé en ce que** les lignées isotransgéniques obtenues sont des lignées élites commerciales.

9. Procédé selon l'une des revendications 1 à 8, **caractérisé en ce qu'**il permet l'introgression de plusieurs caractères transgéniques dans une plante sans addition de fragments liés au transgène pouvant faire l'objet d'un fardeau génétique.

10. Procédé selon l'une des revendications précédentes permettant de cibler le génome parent receveur d'un ADN-T, fragment d'ADN contenant un transgène et les séquences permettant son expression, transféré et intégré dans le génome de l'hôte au cours de la transformation, après transformation d'un hybride, comprenant l'identification des séquences génomiques adjacentes audit ADN-T inséré.

## Patentansprüche

1. Verfahren für die Herstellung von isotransgenen Pflanzenlinien mit den folgenden Schritten:
a) Transformation von pflanzlichen Zellen eines Pflanzenhybrids, der durch Kreuzen von zwei Elternlinien, einer interessierenden Linie und einer für die Transformation geeigneten Linie, gewonnen wurde, mit einem T-DNA-haltigen Vektor, wobei das DNA-Segment ein Transgen und die Sequenzen, die seine Expression gestatten, enthält, während der Transformation in das Wirtsgenom eingebracht und integriert wird;
b) Selektion von primären Hybridtransformanten, die diese T-DNA nur in das Genom der interessierenden Linie integriert enthalten;
c) Rückkreuzen der in b) selektierten Primärtransformanten mit der interessierenden Elternlinie und Selektion von Einzelpflanzen aus diesen Rückkreuzungen, bis man zu isotransgenen Linien gelangt.

2. Verfahren nach Anspruch 1, **dadurch gekennzeichnet, daß** der Schritt der Selektion der primären Hybridtransformanten darin besteht, daß die der insertierten T-DNA benachbarten Genomsequenzen identifiziert werden, um das Elterngenom, das diese T-DNA empfangen hat, zu bestimmen.

3. Verfahren nach Anspruch 2, bei dem die Bestimmung des Elterngenoms, das diese T-DNA empfangen hat, ausgehend von den der T-DNA benachbarten Genomsequenzen mit einer RFLP-Technik oder einer Sequenzierungsmethode durchgeführt wird.

4. Verfahren nach einem der Ansprüche 1 bis 3, bei dem bei der ersten Rückkreuzung von c) diejenigen Einzelpflanzen selektiert werden, bei denen das Chromosom, das die T-DNA empfangen hat, einen vollständig dem Typ der interessierenden Linie entsprechenden Genotyp beibehalten hat, und bei denen das Verhältnis zwischen interessierendem Genom und Gesamtgenom mindestens 75% beträgt.

5. Verfahren nach einem der vorhergehenden Ansprüche, **dadurch gekennzeichnet, daß** es einen letzten Schritt mit einer Kreuzung zwischen der erfindungsgemäßen isotransgenen Linie und einer anderen interessierenden Linie, insbesondere einer anderen isotransgenen Linie, die ein anderes Transgen enthält, umfaßt, wodurch man zu einer Hybridlinie gelangt.

6. Verfahren nach einem der vorhergehenden Ansprüche, **dadurch gekennzeichnet, daß** die pflanzlichen Zellen von einer Hauptkulturart aus der Gruppe Mais, Weizen, Raps, Sonnenblume, Erbse, Sojabohne, Gerste, Gemüseart oder Blumenart stammen.

7. Verfahren nach einem der vorhergehenden Ansprüche, **dadurch gekennzeichnet, daß** die T-DNA insbesondere eine Nukleotidsequenz enthält, die für ein Protein, das landwirtschaftliche Eigenschaften und/oder Resistenz gegen Krankheiten vermittelt, codiert.

8. Verfahren nach einem der vorhergehenden Ansprüche, **dadurch gekennzeichnet, daß** es sich bei den erhaltenen isotransgenen Linien um kommerzielle Elitelinien handelt.

9. Verfahren nach einem der Ansprüche 1 bis 8, **dadurch gekennzeichnet, daß** es die Einbringung von mehreren transgenen Merkmalen in eine Pflanze ohne die Hinzufügung von Fragmenten, die mit dem Transgen verbunden sind und die eine genetische Belastung darstellen können, gestattet.

10. Verfahren nach einem der vorhergehenden Ansprüche, das das Targeting des Elterngenoms, das eine T-DNA empfangen hat, wobei das DNA-Segment ein Transgen und die Sequenzen, die seine Expression gestatten, enthält, während der Transformation in das Wirtsgenom eingebracht und integriert wird, nach der Transformation eines Hybrids gestattet und das die Identifikation von den der insertierten T-DNA benachbarten Genomsequenzen beinhaltet.

## Claims

1. Method for producing isotransgenic plant lines, comprising the following steps of:
a) transforming the plant cells of a plant hybrid consisting of the crossing of two parental lines, a line of interest and a line suited to transformation, with a vector carrying a T-DNA, DNA fragment containing a transgene and the sequences which allow its expression, which is transferred and integrated into the host genome during transformation;
b) selecting the hybrid primary transformants which have integrated said T-DNA only into the genome of the line of interest;
c) backcrossing, with the parental line of interest, said primary transformants selected in b), and selecting the individuals derived from these backcrosses until isotransgenic lines are produced.

2. Method according to Claim 1, **characterized in that** the step for selecting the hybrid primary transformants consists in identifying the genomic sequences adjacent to the T-DNA inserted, in order to determine the parent genome which has received said T-DNA.

3. Method according to Claim 2, in which the determination of the parent genome which has received said T-DNA, using said genomic sequences adjacent to the T-DNA, is carried out according to an RFLP technique or a sequencing method.

4. Method according to one of Claims 1 to 3, in which the individuals in which the chromosome which has received the T-DNA has conserved a genotype entirely of the line of interest type, and which have a genome of interest to entire genome ratio of at least 75%, are selected from the first backcross in c).

5. Method according to one of the preceding claims, **characterized in that** it comprises a subsequent step of crossing between the isotransgenic line according to the invention and another line of interest, in particular another isotransgenic line containing a different transgene, for producing a hybrid line.

6. Method according to one of the preceding claims, **characterized in that** the plant cells originate from a large crop species chosen from maize, wheat, rapeseed, sunflower, pea, soybean and barley, or from a vegetable or floral species.

7. Method according to one of the preceding claims, **characterized in that** the T-DNA comprises in particular a nucleotide sequence encoding a protein which confers agronomic properties and/or properties of resistance to diseases.

8. Method according to one of the preceding claims, **characterized in that** the isotransgenic lines produced are commercial elite lines.

9. Method according to one of Claims 1 to 8, **characterized in that** it allows the introgression of several transgenic characteristics into a plant, without adding fragments linked to the transgene which may be the subject of a genetic burden.

10. Method according to one of the preceding claims, which makes it possible to target the parent genome which has received a T-DNA, DNA fragment containing a transgene and the sequences which allow its expression, which is transferred and integrated into the host genome during transformation, after transformation of a hybrid, comprising the identification of the genomic sequences adjacent to said T-DNA inserted.


### LISTE DE SEQUENCES

<110> RHOBIO
<120> Procédé d'obtention de lignées isotransgéniques
<130>
<140>
   <141>
<160> 30
<170> PatentIn Ver. 2.1
<210> 1
   <211> 22
   <212> ADN
   <213> synthetic construct
<400> 1
   atcatcctgt gacggaactt tg 22
<210> 2
   <211> 22
   <212> ADN
   <213> synthetic construct
<400> 2
   atcatcctgt gacggaactt tg 22
<210> 3
   <211> 20
   <212> ADN
   <213> synthetic construct
<400> 3
   gctcggcaca aaatcaccac 20
<210> 4
   <211> 22
   <212> ADN
   <213> synthetic construct
<400> 4
   catagttctc aagatcgaca gc 22
<210> 5
   <211> 29
   <212> ADN
   <213> synthetic construct
<400> 5
   gcaggcatgc aagcttcagc tgctcgatc 29
<210> 6
   <211> 28
   <212> ADN
   <213> synthetic construct
<400> 6
   ccgcaatgtg ttattaagtt gtctaagc 28
<210> 7
   <211> 28
   <212> ADN
   <213> synthetic construct
<400> 7
   caggcatgca agcttcagct gctcgatc 28
<210> 8
   <211> 27
   <212> ADN
   <213> synthetic construct
<400> 8
   ctgcaggcat gcaagcttca gctgctc 27
<210> 9
   <211> 27
   <212> ADN
   <213> synthetic construct
<400> 9
   tgcaggcatg caagcttcag ctgctcg 27
<210> 10
   <211> 27
   <212> ADN
   <213> synthetic construct
<400> 10
   aggcatgcaa gcttcagctg ctcgatc 27
<210> 11
   <211> 27
   <212> ADN
   <213> synthetic construct
<400> 11
   cagtacatta aaaacgtccg caatgtg 27
<210> 12
   <211> 27
   <212> ADN
   <213> synthetic construct
<400> 12
   acgtccgcaa tgtgttatta agttgtc 27
<210> 13
   <211> 26
   <212> ADN
   <213> synthetic construct
<400> 13
   tgcaggcatg caagcttcag ctgctc 26
<210> 14
   <211> 26
   <212> ADN
   <213> synthetic construct
<400> 14
   ggcatgcaag cttcagctgc tcgatc 26
<210> 15
   <211> 26
   <212> ADN
   <213> synthetic construct
<400> 15
   cgtccgcaat gtgttattaa gttgtc 26
<210> 16
   <211> 29
   <212> ADN
   <213> synthetic construct
<400> 16
   atgatcagat tgtcgtttcc cgccttcag 29
<210> 17
   <211> 29
   <212> ADN
   <213> synthetic construct
<400> 17
   gactccctta attctccgct catgatcag 29
<210> 18
   <211> 29
   <212> ADN
   <213> synthetic construct
<400> 18
   gcggttctgt cagttccaaa cgtaaaacg 29
<210> 19
   <211> 29
   <212> ADN
   <213> synthetic construct
<400> 19
   tgcggttctg tcagttccaa acgtaaaac 29
<210> 20
   <211> 28
   <212> ADN
   <213> synthetic construct
<400> 20
   tgatcagatt gtcgtttccc gccttcag 28
<210> 21
   <211> 28
   <212> ADN
   <213> synthetic construct
<400> 21
   actcccttaa ttctccgctc atgatcag 28
<210> 22
   <211> 28
   <212> ADN
   <213> synthetic construct
<400> 22
   cggttctgtc agttccaaac gtaaaacg 28
<210> 23
   <211> 28
   <212> ADN
   <213> synthetic construct
<400> 23
   gcggttctgt cagttccaaa cgtaaaac 28
<210> 24
   <211> 27
   <212> ADN
   <213> synthetic construct
<400> 24
   gatcagattg tcgtttcccg ccttcag 27
<210> 25
   <211> 27
   <212> ADN
   <213> synthetic construct
<400> 25
   ctcccttaat tctccgctca tgatcag 27
<210> 26
   <211> 27
   <212> ADN
   <213> synthetic construct
<400> 26
   tcatcggcgg gggtcataac gtgactc 27
<210> 27
   <211> 27
   <212> ADN
   <213> synthetic construct
<400> 27
   ggttctgtca gttccaaacg taaaacg 27
<210> 28
   <211> 27
   <212> ADN
   <213> synthetic construct
<400> 28
   cggttctgtc agttccaaac gtaaaac 27
<210> 29
   <211> 26
   <212> ADN
   <213> synthetic construct
<400> 29
   atcagattgt cgtttcccgc cttcag 26
<210> 30
   <211> 26
   <212> ADN
   <213> synthetic construct
<400> 30
   tcccttaatt ctccgctcat gatcag 26